(19) Europäisches Patentamt
European Patent Office
Office européen des brevets

(11) EP 1 781 245 B1

(12) **EUROPEAN PATENT SPECIFICATION**

(45) Date of publication and mention of the grant of the patent:
**14.11.2012 Bulletin 2012/46**

(21) Application number: **05755745.6**

(22) Date of filing: **28.06.2005**

(51) Int Cl.:
***A61Q 5/04*** *(2006.01)* ***A61K 8/46*** *(2006.01)*

(86) International application number:
**PCT/JP2005/012274**

(87) International publication number:
**WO 2006/001536 (05.01.2006 Gazette 2006/01)**

(54) **METHOD FOR PERMANENT HAIR PROCESSING**

VERFAHREN FÜR DIE PERMANENTE HAARBEARBEITUNG

PROCEDE POUR LA TRANSFORMATION PERMANENTE DES CHEVEUX

(84) Designated Contracting States:
**AT BE BG CH CY CZ DE DK EE ES FI FR GB GR HU IE IS IT LI LT LU MC NL PL PT RO SE SI SK TR**

(30) Priority: **28.06.2004 JP 2004190330**

(43) Date of publication of application:
**09.05.2007 Bulletin 2007/19**

(73) Proprietor: **Showa Denko K.K.**
**Tokyo 105-8518 (JP)**

(72) Inventors:
- **SHIBUYA, Akira**
**Kawasaki-ku, Kawasaki-shi, Kanagawa 210-0865 (JP)**
- **SAITO, Makoto**
**Kawasaki-ku, Kawasaki-shi, Kanagawa 210-0865 (JP)**
- **ISHII, Hiroshi**
**Kawasaki-ku, Kawasaki-shi, Kanagawa 210-0865 (JP)**

(74) Representative: **Strehl Schübel-Hopf & Partner**
**Maximilianstrasse 54**
**80538 München (DE)**

(56) References cited:
**US-A- 3 525 761 US-A- 3 880 174**
**US-A- 5 720 944 US-A- 5 720 945**

- **VOSS JG: "Skin sensitization by mercaptans of low molecular weight" THE JOURNAL OF INVESTIGATIVE DERMATOLOGY, vol. 31, no. 5, 1958, pages 273-279, XP009053277**
- **PATENT ABSTRACTS OF JAPAN vol. 1995, no. 01, 28 February 1995 (1995-02-28) & JP 06 279396 A (NIPPON SHOKUBAI CO LTD), 4 October 1994 (1994-10-04)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 781 245 B1

## Description

### FIELD OF THE INVENTION

The present invention relates to a method for permanent hair processing that contains a mercaptocarboxylic acid derivative and enables hair processing in an acidic to neutral region.

### BACKGROUND OF THE INVENTION

So-called keratin reducing substances such as thioglycolic acids and salts of cysteine and acetylcysteine have been used to give hair a permanent shape. These keratin reducing substances provide practical performance of permanent hair processing under alkaline conditions, and therefore most permanent solutions are made alkaline with a pH around 9.5. However, the alkaline permanent solutions are known to damage the hair and scalp. To solve such problems, keratin reducing substances usable with the pH being in a neutral to slightly acidic region have been studied.

For example, monoglycerol thioglycolates have been studied to improve the odor problem with low-boiling thioglycolates having a hydroxyl group in the alcohol portion, such as methyl thioglycolate, as well as to obtain performance at neutral pH. (Patent Document 1)

The monoglycerol thioglycolates are liquid to permit excellent handling properties and have less odor. However, there are some reports of sensitizing potential that is probably attributed to the hydroxyl group in the structure, and none has reached a practical level.

Meanwhile, mercaptoglycolic acid amide derivatives and mercaptolactic acid amide derivatives have been studied to solve the problems of damaged skin by the monoglycerol thioglycolates. (Patent Documents 2 and 3)

However, mercaptocarboxylic acid amides are known to cause skin irritation, and therefore it is feared that the mercaptocarboxylic acid amide derivatives have a similar sensitizing potential too. Furthermore, there is concern that insufficient purification and liberation of raw material amine during storage will lead to sensitization and skin irritation.

Moreover, a permanent waving method has been disclosed in which hair is treated with part of an agent based on a mercaptocarboxylic acid salt and thereafter with the remaining agent mixed with a mercaptocarboxylic acid ester. (Patent Document 4)

This method, however, is not practical because the mercaptocarboxylic acid ester, which is used as an auxiliary, has bad stability and must be mixed every time of use.

Patent Document 1: JP-A-H08-291031

Patent Document 2: JP-A-2000-507272

Patent Document 3: JP-A-2003-528901

Patent Document 4: JP-A-S50-111242

US 3,880,174 teaches to use mercaptocarboxylic acid esters in combination with mercaptocarboxylic acid salts for permanent hair processing.

### DISCLOSURE OF THE INVENTION

It is an object of the invention to provide a method for permanent hair processing that can shape the hair at a neutral to slightly acidic pH. The method is defined in claim 1.

The present inventors studied diligently and have arrived at an alkoxy mercaptocarboxylate obtained by reacting a glycol monoalkyl ether with a mercaptocarboxylic acid. It has been found that the alkoxy mercaptocarboxylate when used as a keratin reducing agent permits reduction of odor and sensitizing potential inherent to esters, has no sensitizing potential inherent to mercapto-containing amides or attributed to impurities, and shows sufficient penetration properties. The present invention has been completed based on the finding.

The invention pertains to a process using the agent as in (I) to (VI).

(I) An agent for permanent hair processing comprising at least one of compounds represented by the following formula (1) and/or formula (2) :

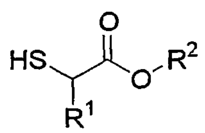

(1)

wherein $R^1$ is a hydrogen atom or an alkyl group of 1 to 5 carbon atoms, $R^2$ is an alkoxyalkyl group having a total

of 3 to 15 carbon atoms, and the alkylene portion of $R^2$ may contain an ether bond(s);

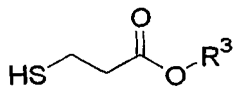
(2)

wherein $R^3$ is an alkoxyalkyl group having a total of 3 to 15 carbon atoms, and the alkylene portion of $R^3$ may contain an ether bond (s).

(II) The agent for permanent hair processing as described in (I), wherein the agent contains at least one compound of the formula (1) in which $R^1$ is a hydrogen atom, a methyl group, an ethyl group or a propyl group, and $R^2$ is an alkoxyalkyl group including an alkoxy group of 1 to 4 carbon atoms and an alkylene group of 1 to 8 carbon atoms (the alkylene group of $R^2$ may contain an ether bond(s)).

(III) The agent for permanent hair processing as described in (I), wherein the agent contains at least one compound of the formula (2) in which $R^3$ is an alkoxyalkyl group including an alkoxy group of 1 to 4 carbon atoms and an alkylene group of 1 to 8 carbon atoms (the alkylene group of $R^3$ may contain an ether bond(s)).

(IV) The agent for permanent hair processing as described in (I), wherein the agent contains the compound (s) represented by the formula (1) and/or formula (2) in an amount of 0.5 to 30% in terms of reducing power of thioglycolic acid.

(V) The agent for permanent hair processing as described in (I) or (IV), wherein the agent contains two or more compounds represented by the formula (1) and/or formula (2).

(VI) The agent for permanent hair processing as described in (I), wherein the agent has a pH adjusted in the range of 6 to 8.5.

The agent for permanent hair processing that contains an alkoxyalkyl mercaptocarboxylate shows excellent permanent processing performance even in a neutral to slightly acidic pH region. The agent is capable of superior permanent processing performance even when it contains the alkoxyalkyl mercaptocarboxylate at low concentrations. The permanent processing agent of the invention is therefore highly useful in the permanent hair processing.

## BRIEF DESCRIPTION OF THE DRAWINGS

Fig. 1 is a diagram showing the results of gas chromatography mass spectrometry of the compound prepared in Synthetic Example 1; and

Fig. 2 is a diagram showing the results of infrared absorption spectrometry of the compound prepared in Synthetic Example 1.

## PREFERRED EMBODIMENTS OF THE INVENTION

Hereinbelow, the present invention will be described in detail.

The agent for permanent hair processing to be used in the method according to the invention contains at least one alkoxyalkyl mercaptocarboxylate represented by the formula (1) and/or the formula (2) below.

[Compound (1)]

First, the compound (1) will be discussed. It has the following formula (1):

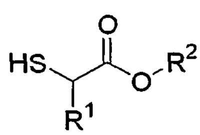
(1)

wherein $R^1$ is a hydrogen atom or an alkyl group of 1 to 5 carbon atoms, $R^2$ is an alkoxyalkyl group having a total of 3 to 15 carbon atoms, and the alkylene portion of $R^2$ may contain an ether bond (s).

In the formula (1), $R^1$ is a hydrogen atom or an alkyl group of 1 to 5 carbon atoms, and is preferably a hydrogen atom, a methyl group, an ethyl group or a propyl group.

$R^2$ in the formula (1) is a combination of a terminal alkoxy group and an alkylene group bonded thereto. The alkylene group may have its carbon atom(s) substituted with an oxygen atom(s) to form an ether bond(s). $R^2$ preferably has a total of 3 to 15 carbon atoms. The alkoxy group preferably has 1 to 4 carbon atoms, while the alkylene group preferably has 1 to 8 carbon atoms.

Examples of $R^2$ include 2-methoxyethyl, 2-ethoxyethyl, 2-propoxyethyl, 2-isopropoxyethyl, 2-butoxyethyl, 2-isobutoxyethyl, 2-tert-butoxyethyl, 5-methoxy-3-oxapentyl, 5-ethoxy-3-oxapentyl, 5-propoxy-3-oxapentyl, 5-isopropoxy-3-oxapentyl, 5-butoxy-3-oxapentyl, 5-isobutoxy-3-oxapentyl, 5-tert-butoxy-3-oxapentyl, 8-methoxy-3,6-dioxaoctyl, 8-ethoxy-3,6-dioxaoctyl, 8-propoxy-3, 6-dioxaoctyl, 8-isopropoxy-3,6-dioxaoctyl, 8-butoxy-3,6-dioxaoctyl, 8-isobutoxy-3,6-dioxaoctyl, 8-tert-butoxy-3,6-dioxaoctyl, 2-methoxy-1-methylethyl, 2-ethoxy-1-methylethyl, 2-propoxy-1-methylethyl, 2-isopropoxy-1-methylethyl, 2-butoxy-1-methylethyl, 2-isobutoxy-1-methylethyl, 2-tert-butoxy-1-methylethyl, 5-methoxy-1,4-dimethyl-3-oxapentyl, 5-ethoxy-1,4-dimethyl-3-oxapentyl, 5-propoxy-1,4-dimethyl-3-oxapentyl, 5-isopropoxy-1,4-dimethyl-3-oxapentyl, 5-butoxy-1,4-dimethyl-3-oxapentyl, 5-isobutoxy-1,4-dimethyl-3-oxapentyl, 5-tert-butoxy-1,4-dimethyl-3-oxapentyl, 8-methoxy-1,4,7-trimethyl-3,6-dioxaoctyl, 8-ethoxy-1,4,7-trimethyl-3,6-dioxaoctyl, 8-propoxy-1,4,7-trimethyl-3,6-dioxaoctyl, 8-isopropoxy-1,4,7-trimethyl-3,6-dioxaoctyl, 8-butoxy-1,4,7-trimethyl-3,6-dioxaoctyl, 8-isobutoxy-1,4,7-trimethyl-3,6-dioxaoctyl and 8-tert-butoxy-1,4,7-trimethyl-3,6-dioxaoctyl. Of these, 2-methoxyethyl, 2-ethoxyethyl, 2-methoxy-1-methylethyl and 2-ethoxy-1-methylethyl are preferred.

Preferred compounds of the formula (1) include 2-methoxyethyl thioglycolate, 2-ethoxyethyl thioglycolate, 2-methoxy-1-methylethyl thioglycolate, 2-ethoxy-1-methylethyl thioglycolate, 2-methoxyethyl thiolactate, 2-ethoxyethyl thiolactate, 2-methoxy-1-methylethyl thiolactate and 2-ethoxy-1-methylethyl thiolactate.

[Compound (2)]

Next, the compound (2) will be described. It has the following formula (2):

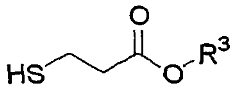
(2)

wherein $R^3$ is an alkoxyalkyl group having a total of 3 to 15 carbon atoms, and the alkylene portion of $R^3$ may contain an ether bond(s).

$R^3$ in the formula (2) is a combination of a terminal alkoxy group and an alkylene group bonded thereto. The alkylene group may have its carbon atom(s) substituted with an oxygen atom(s) to form an ether bond(s). $R^3$ preferably has a total of 3 to 15 carbon atoms. The alkoxy group preferably has 1 to 4 carbon atoms, while the alkylene group preferably has 1 to 8 carbon atoms.

Examples of $R^3$ include those mentioned for the functional group $R^2$ of the compound (1). Preferred are 2-methoxyethyl, 2-ethoxyethyl, 2-methoxy-1-methylethyl and 2-ethoxy-1-methylethyl.

Preferred compounds of the formula (2) include 2-methoxyethyl mercaptopropionate, 2-ethoxyethyl mercaptopropionate, 2-methoxy-1-methylethyl mercaptopropionate and 2-ethoxy-1-methylethyl mercaptopropionate.

[Production of compounds (1) and (2)]

The alkoxyalkyl mercaptocarboxylates represented by the formulae (1) and (2) are preferably obtained by dehydration reaction or ester exchange reaction in which a mercaptocarboxylic acid or an ester thereof (hereinafter, the mercaptocarboxylic acid or ester) is mixed with a alkoxyalkyl alcohol under heating in the presence of an acid catalyst.

The mercaptocarboxylates suitable for the reaction include methyl mercaptocarboxylate and ethyl mercaptocarboxylate.

The alkoxyalkyl alcohol is generally used in an equimolar amount to the mercaptocarboxylic acid or ester. When the alkoxyalkyl alcohol serves also as a solvent, it is used at least in an equimolar amount, and preferably in a 1-10 fold equimolar amount to the mercaptocarboxylic acid or ester. When the alkoxyalkyl alcohol is used in excess of the mercaptocarboxylic acid or ester, the unreacted raw material is preferably recovered and reused.

The acid catalysts include hydrochloric acid, sulfuric acid, nitric acid, toluenesulfonic acid, and ion-exchange resins having a sulfuric acid residue. The amount of the acid catalyst is preferably in the range of 0 to 10% by mass relative to the mercaptocarboxylic acid or ester.

The esterification reaction takes place while generating a low-molecular alcohol and/or water. Removing such by-products during the reaction improves the yield. Methods for removing the by-products include thermal evaporation, azeotropic evaporation, use of molecular sieves and dehydration with sodium sulfate. After the low-boiling alcohol, water and the raw material alcohol and mercaptocarboxylic acid or ester are removed, the resultant solution of the alkoxyalkyl

mercaptocarboxylate further contains high-boiling by-products such as 8-methoxy-3,6-dioxaoctyl thioglycolate. Although the solution can be used directly as an agent for permanent hair processing in order to use effectively such compounds, it is preferably used after distilled under reduced pressure to purify the alkoxyalkyl mercaptocarboxylate.

The product purified by distillation preferably contains the alkoxyalkyl mercaptocarboxylate having a purity of at least 99% by mass relative to all the mercapto-containing compounds in the product. The purified product may contain the raw material monoalkoxyalkyl alcohol at 50% by mass or less, and preferably 20% by mass or less. The purified product is a liquid in most cases, and is usable directly as a permanent processing agent.

When the raw material used is the mercaptocarboxylate, unreacted mercaptocarboxylate is preferably removed from the product to a concentration of 1% by mass or less, and still preferably 0.1% by mass or less, because it has a strong odor. When the raw material used is the mercaptocarboxylic acid, unreacted mercaptocarboxylic acid may remain in the product.

[Agent for permanent hair processing]

The agent for permanent hair processing contains at least one alkoxyalkyl mercaptocarboxylate of the formula (1) and/or at least one alkoxyalkyl mercaptocarboxylate of the formula (2).

The alkoxyalkyl mercaptocarboxylates of the formula (1) or (2) may be used singly or in combination of two or more kinds. The compounds of the formulae (1) and (2) may be used in combination.

The agent for permanent hair processing preferably contains the alkoxyalkyl mercaptocarboxylate of the formula (1) and/or the formula (2) in an amount of 0.5 to 30%, more preferably 1 to 15%, and most preferably 2 to 10% in terms of reducing power of thioglycolic acid.

When the amount in terms of reducing power of thioglycolic acid is less than 0.5%, the agent often cannot provide any perming performance. The amount exceeding 15% may lead to undesirable results, such as excessively waved hair and damaged hair by promoted partial removal of cuticles.

The amount in terms of reducing power of thioglycolic acid is a notation of the concentration of keratin reducing substance that is determined by the following procedure.

Precisely 10 ml of a sample is placed into a 100 ml graduated flask. Purified water conforming to Japanese Standards of Cosmetic Ingredients (hereinafter, simply referred to as water) is added to make the total amount 100 ml. This mixture is used as a testing solution.

Precisely 20 ml of the testing solution is combined with 50 ml of water and 5 ml of 30% sulfuric acid. The mixture is subjected to mild heating and is boiled for 5 minutes. After cooled, the mixture is titrated with a 0.1 N iodine solution to determine the consumption (Aml) (indicator: 3 ml of starch test solution).

From the titration result, the concentration in terms of thioglycolic acid is calculated by the following equation:

$$\text{Concentration (\%) of reducing substance (in terms of thioglycolic acid)} = 0.4606 \times A$$

Formulations of the permanent hair processing agent of the invention are not particularly limited as long as the agent consists of the alkoxyalkyl mercaptocarboxylate represented by the formula (1) or (2). Examples of the formulations include liquids, foams, gels, creams and pastes. Depending on the formulation, the agent may be used in the form of liquid type, spray type, aerosol type, cream type or gel type.

The agent may contain various additives for improved performance of hair processing. Suitable additives include swelling agents, penetration accelerators, buffers, oily medicines, thickeners, hair protective agents, wetting agents, emulsifying agents, pH adjusters, perfumes, colorants, stabilizers and odor masking agents.

The swelling agents and penetration accelerators include ethanol, propanol, isopropanol, 1,2-propyleneglycol, 1,3-butanediol, glycerol, ethylcarbitol, benzyl alcohol, benzyloxyethanol, urea and 2-methylpyrrolidone.

The buffers include inorganic buffers, and buffers containing basic amino acid, such as ammonium salt-arginine and ammonium salt-lysine.

The oily medicines include paraffin, liquid paraffin, beeswax, squalane, jojoba oil, olive oil, ester oil, triglyceride, vaseline and lanolin.

The thickeners include carboxymethylcellulose, carboxyvinyl polymers, hydroxyethylcellulose, hydroxypropylcellulose, xanthan gum, carrageenan, alginic acid salts, pectin, tragacanth gum, higher alcohols such as lauryl alcohol, cetyl alcohol, stearyl alcohol, isostearyl alcohol, oleyl alcohol and behenyl alcohol, kaolin, fatty acids such as lauric acid, myristic acid, palmitic acid, stearic acid, behenic acid, oleic acid, undecylic acid and isostearic acid, and vaseline.

The hair protective agents include collagen and keratin hydrolysates and derivatives thereof.

The wetting agents and emulsifying agents include glycerol, diglycerol, propyleneglycol, dipropyleneglycol, 1,3-butanediol, sorbitol, plant extracts, vitamins, hyaluronic acid salts, chondroitin sulfate, cationic, anionic, amphoteric and nonionic surfactants, ether-based nonionic surfactants such as polyoxyethylene oleylether, polyoxyethylene stearylether, polyoxyethylene cetylether, polyoxyethylene octylphenylether, polyoxyethylene dodecylphenylether and polyoxyethylene nonylether, dimethylpolysiloxane, methylphenylpolysiloxane, and silicon derivatives such as amino-modified silicon oils, alcohol-modified silicon oils, fluorine-modified silicon oils, polyether-modified silicon oils and alkyl-modified silicon oils.

The pH adjusters include hydrochloric acid, organic acids such as citric acid, malic acid, lactic acid, succinic acid and oxalic acid, sodium salts of the acids, and alkaline agents such as ammonia, diethanolamine, triethanolamine, sodium hydroxide, potassium hydroxide, sodium carbonate, sodium hydrogencarbonate, potassium carbonate and potassium hydrogencarbonate.

The stabilizers for preventing excessive reduction include disulfides of reduced compounds and dithiodiglycolic acid.

The pH of the agent is 6 to 8.5. The agent having this pH seldom causes irritation to the skin and does not damage the hair and scalp. The agent of the invention can exhibit practical performance of permanent processing at the above pH range.

The above pH can be achieved by adding the pH adjuster to the agent.

[Permanent processing method]

The agent for permanent hair processing that consists of the ingredients described above is used according to claim 1. In the invention, the permanent processing treatments include permanent wave treatment, permanent wave removal treatment and frizz straightening treatment.

(1) The agent containing the alkoxyalkyl mercaptocarboxylate in this invention is applied to the hair, and the wet hair is rolled onto rods for shaping.
The frizz straightening treatment does not involve rods.
The hair may be first wetted with water, rolled onto rods, and then given the agent.
(2) The hair wetted with the agent is allowed to stand, preferably at elevated temperatures of 30 to 40°C.
(3) The hair is oxidized with a composition containing an oxidant, and is tightened.
(4) The rods are removed from the tight hair, and the hair is rinsed, shampooed and dried.

The oxidant used in (3) may be a common oxidant, with examples including approximately 3 to 8% aqueous solution of sodium bromate, and diluted solutions of hydrogen peroxide and sodium perborate.

## EXAMPLES

The present invention will be described in more detail with reference to the following examples, but it should be construed that the invention is in no way limited to the examples.

[Synthetic Example 1]

<u>Synthesis of 2-methoxyethyl thioglycolate (TGE-1)</u>

A 1000-ml four-necked flask equipped with a thermometer and a cooling tube was charged with 300 g of methyl thioglycolate, 320 g of 2-methoxyethanol and 3.6 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by high performance liquid chromatography (HPLC) to determine the yield, which was found to be 42% from calculation of the concentration of the objective compound in the reaction liquid.

Subsequently, the reaction liquid was directly concentrated and distilled under reduced pressure (b.p. 65°C / 0. 6 kPa). This purification provided 123 g of 2-methoxyethyl thioglycolate (TGE-1).

The concentration of the components in the reaction liquid was analyzed under the following conditions.

HPLC analysis

Columns: Shodex™ NN-G (guard column) and NN-814 manufactured by Showa Denko K.K.

Detectors: RI detector and UV detector (210 nm)

Eluent: 0.1% phosphoric acid plus 0.008 M aqueous monobasic potassium phosphate solution
Flow rate: 1.0 ml/min.
Injection amount: 20 μl
TGE-1 was identified by gas chromatography mass spectrometry (GC-MS) and infrared (IR) absorption spectrometry. The analysis charts are shown in Figs. 1 and 2. The analysis results are given below.
GC-MS: 118, 77, 58, 45, 31
IR (Zn-Ce): 2932, 2889, 1732, 1408, 1123, 1099, 1033, 865
The identification employed the following equipment and conditions:

GC-MS analysis

Equipment: GC-17A and GCMS-QP5000 manufactured by Shimadzu Corporation
Column: TC-5 (inner diameter: 0.25 mm, membrane thickness: 0. 25 μm, length: 30 m) manufactured by GL Sciences
Carrier Gas: Helium
flow rate: 0.87 cc/min.
Split ratio: 80:1
Heating program: 70°C (5 min.) →10°C/min.→250°C (10 min.)
Injection amount: 1 μl

IR analysis

Equipment: FT/IR-7300 manufactured by JASCO Corporation
Methods: KBr method and ATR method (Zn-Ce)

[Synthetic Example 2]

Synthesis of 2-ethoxyethyl thioglycolate (TGE-2)

A 1000-ml four-necked flask equipped with a thermometer and a cooling tube was charged with 300 g of methyl thioglycolate, 374 g of 2-ethoxyethanol and 3.6 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by gas chromatography (GC) to determine the yield, which was found to be 44% from calculation of the concentration of the objective compound in the reaction liquid.
Subsequently, the reaction liquid was directly concentrated and distilled under reduced pressure (b.p. 99 to 103°C / 2.1 kPa). This purification provided 89 g of 2-ethoxyethyl thioglycolate (TGE-2).
The concentration of the components in the reaction liquid was analyzed under the following conditions.

GC analysis

Column: TC-5 (inner diameter: 0.25 mm, membrane thickness: 0.25 μm, length: 30 m) manufactured by GL Sciences
Carrier Gas: Helium
flow rate: 0.87 cc/min.
Split ratio: 80:1
Heating program: 70°C (5 min.) →10°C/min.→250°C (10 min.)
Injection amount: 1 μl
The results of TGE-2 identification are given below.
GC-MS: 91, 72, 59, 45, 31
IR (Zn-Ce) : 2976, 2870, 1733, 1275, 1117, 1038, 961, 863

[Synthetic Example 3]

Synthesis of 2-butoxyethyl thioglycolate (TGE-3)

A 1000-ml four-necked flask equipped with a thermometer and a cooling tube was charged with 300 g of methyl thioglycolate, 489 g of 2-n-butoxyethanol and 3.6 g of 95% sulfuric acid, followed by stirring at 80°C for 7 hours. During

the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 35% from calculation of the concentration of the objective compound in the reaction liquid.

Subsequently, the reaction liquid was directly concentrated and distilled under reduced pressure (b.p. 84°C / 0.5 kPa). This purification provided 136 g of 2-butoxyethyl thioglycolate (TGE-3).

The results of TGE-3 identification are given below.

GC-MS: 145, 134, 118, 101, 85, 64

IR (Zn-Ce): 2957, 2935, 2869, 1736, 1456, 1275, 1120, 1031

[Synthetic Example 4]

Synthesis of 5-methoxy-3-oxapentyl thioglycolate (TGE-4)

A 1000-ml four-necked flask equipped with a thermometer and a cooling tube was charged with 300 g of methyl thioglycolate, 339 g of diethylene glycol monomethyl ether and 3.3 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 32% from calculation of the concentration of the objective compound in the reaction liquid.

Subsequently, the reaction liquid was directly concentrated and distilled under reduced pressure (b.p. 101°C / 0.6 kPa). This purification provided 136 g of 5-methoxy-3-oxapentyl thioglycolate (TGE-4).

The results of TGE-4 identification are given below. GC-MS: 119, 75, 59, 45, 31

IR (Zn-Ce) : 2879, 1733, 1453, 1277, 1105, 1043, 961, 849

[Synthetic Example 5]

Synthesis of 5-ethoxy-3-oxapentyl thioglycolate (TGE-5)

A 1000-ml four-necked flask equipped with a thermometer and a cooling tube was charged with 300 g of methyl thioglycolate, 409 g of diethylene glycol monoethyl ether and 3.0 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 31% from calculation of the concentration of the objective compound in the reaction liquid.

Subsequently, the reaction liquid was directly concentrated and distilled under reduced pressure (b.p. 98°C / 0.6 kPa). This purification provided 122 g of 5-ethoxy-3-oxapentyl thioglycolate (TGE-5).

The results of TGE-5 identification are given below.

GC-MS: 162, 135, 118, 86, 72, 59, 45

IR (Zn-Ce): 2869, 1735, 1449, 1276, 1108, 1032

[Synthetic Example 6]

Synthesis of 8-methoxy-3,6-dioxaoctyl thioglycolate (TGE-6)

A 1000-ml four-necked flask equipped with a thermometer and a cooling tube was charged with 300 g of methyl thioglycolate, 462 g of triethylene glycol monoethyl ether and 3.1 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 32% from calculation of the concentration of the objective compound in the reaction liquid.

Subsequently, the reaction liquid was directly concentrated and purified by distilling away low-boiling components under reduced pressure. The purified reaction liquid in the reaction vessel was combined with 500 g of diethylether to give a solution. The solution was then washed with 500 g of water, and the resultant ether solution was washed again with 300 g of water. The ether solution thus obtained was purified by evaporating ether under reduced pressure, and 184 g of 8-methoxy-3,6-dioxaoctyl thioglycolate (TGE-6) was obtained as an oily substance.

The results of TGE-6 identification are given below. GC-MS: 119, 101, 89, 73, 59, 45, 31

IR (Zn-Ce): 2874, 1734, 1277, 1098, 1041, 958, 850

[Synthetic Example 7]

Synthesis of 2-methoxy-1-methylethyl thioglycolate (TGE-7)

A 1000-ml four-necked flask equipped with a thermometer and a cooling tube was charged with 213 g of methyl thioglycolate, 195 g of 1-methoxy-2-propanol and 2.2 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by GC to determine the yield, which was found to be 17% from calculation of the concentration of the objective compound in the reaction liquid.

Subsequently, the reaction liquid was directly concentrated and distilled under reduced pressure (b.p. 102°C / 2.8 kPa). This purification provided 53 g of 2-methoxy-1-methylethyl thioglycolate (TGE-7).

The results of TGE-7 identification are given below.

GC-MS: 132, 116, 103, 86, 75, 59, 45, 31

IR (Zn-Ce): 2983, 1729, 1453, 1274, 1151, 1110, 1097, 1074, 968

[Synthetic Example 8]

Synthesis of 2-ethoxy-1-methylethyl thioglycolate (TGE-8)

A 1000-ml four-necked flask equipped with a thermometer and a cooling tube was charged with 217 g of methyl thioglycolate, 229 g of 1-ethoxy-2-propanol and 2.2 g of 95% sulfuric acid, followed by stirring at 80°C for 7 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 27% from calculation of the concentration of the objective compound in the reaction liquid.

Subsequently, the reaction liquid was directly concentrated and distilled under reduced pressure (b.p. 102°C / 2.8 kPa). This purification provided 59 g of 2-ethoxy-1-methylethyl thioglycolate (TGE-8).

The results of TGE-8 identification are given below.

GC-MS: 132, 119, 103, 87, 75, 59, 45, 31

IR (Zn-Ce): 2978, 2872, 1732, 1275, 1144, 1112, 1063, 960

[Synthetic Example 9]

Synthesis of 8-methoxy-3,6-dioxaoctyl thioglycolate (TGE-9)

A 1000-ml four-necked flask equipped with a thermometer and a cooling tube was charged with 240 g of methyl thioglycolate, 361 g of dipropylene glycol monomethyl ether and 2.4 g of 95% sulfuric acid, followed by stirring at 80°C for 5 hours. During the reaction, the pressure was slightly reduced by means of an aspirator connected with an upper end of the cooling tube, and thereby methanol that occurred with progress of the reaction was distilled away. After the completion of the reaction, part of the reaction liquid was sampled and analyzed by HPLC to determine the yield, which was found to be 25% from calculation of the concentration of the objective compound in the reaction liquid.

Subsequently, the reaction liquid was directly concentrated and distilled under reduced pressure (b.p. 91°C / 0.5 kPa). This purification provided 73 g of 8-methoxy-3,6-dioxaoctyl thioglycolate (TGE-9).

The results of TGE-9 identification are given below.

GC-MS: 177, 148, 133, 73, 59, 45

IR (Zn-Ce): 2935, 1732, 1452, 1274, 1101, 1061, 1024, 963

[Synthetic Example 10]

Synthesis of 2-methoxyethyl thiolactate (TNE-1)

A 200-ml four-necked flask equipped with a thermometer and a cooling tube was charged with 86 g of thiolactic acid, 61 g of 2-methoxyethanol and 1 g of 95% sulfuric acid, followed by stirring at 100°C for 5 hours. During the reaction, the pressure was slightly reduced by means of an aspirator connected with an upper end of the cooling tube, and thereby water that occurred with progress of the reaction was distilled away. After the completion of the reaction, part of the reaction liquid was sampled and analyzed by HPLC to determine the yield, which was found to be 54% from calculation of the concentration of the objective compound in the reaction liquid.

Subsequently, the reaction liquid was directly concentrated and distilled under reduced pressure (b.p. 124°C / 6.4 kPa). This purification provided 70 g of 2-methoxyethyl thiolactate (TNE-1).
The results of TNE-1 identification are given below.
GC-MS: 164, 132, 119, 103, 61, 45, 31
IR (Zn-Ce): 2933, 1736, 1454, 1328, 1175

[Synthetic Example 11]

Synthesis of 2-ethoxyethyl thiolactate (TNE-2)

A 1000-ml four-necked flask equipped with a thermometer and a cooling tube was charged with 202 g of thiolactic acid, 190 g of 2-ethoxyethanol and 2.1 g of 95% sulfuric acid, followed by stirring at 100°C for 3 hours. During the reaction, the pressure was slightly reduced by means of an aspirator connected with an upper end of the cooling tube, and thereby water that occurred with progress of the reaction was distilled away. After the completion of the reaction, part of the reaction liquid was sampled and analyzed by HPLC to determine the yield, which was found to be 56% from calculation of the concentration of the objective compound in the reaction liquid.
Subsequently, the reaction liquid was directly concentrated and distilled under reduced pressure (b.p. 102°C / 2.6 kPa). This purification provided 245 g of 2-ethoxyethyl thiolactate (TNE-2).
The results of TNE-2 identification are given below.
GC-MS: 132, 119, 100, 85, 61, 57
IR (Zn-Ce): 2976, 1733, 1452, 1243, 1169, 1121, 1069, 1028

[Synthetic Example 12]

Synthesis of 2-butoxyethyl thiolactate (TNE-3)

A 1000-ml four-necked flask equipped with a thermometer and a cooling tube was charged with 196 g of thiolactic acid, 253 g of 2-butoxyethanol and 1.9 g of 95% sulfuric acid, followed by stirring at 100°C for 3 hours. During the reaction, the pressure was slightly reduced by means of an aspirator connected with an upper end of the cooling tube, and thereby water that occurred with progress of the reaction was distilled away. After the completion of the reaction, part of the reaction liquid was sampled and analyzed by HPLC to determine the yield, which was found to be 77% from calculation of the concentration of the objective compound in the reaction liquid.
Subsequently, the reaction liquid was directly concentrated and distilled under reduced pressure (b.p. 115°C / 2.8 kPa). This purification provided 313 g of 2-butoxyethyl thiolactate (TNE-3).
The results of TNE-3 identification are given below.
GC-MS: 146, 105, 86, 57
IR (Zn-Ce): 2959, 2934, 1735, 1453, 1326, 1246, 1170, 1124, 1069

[Synthetic Example 13]

Synthesis of 2-ethoxy-1-methylethyl thiolactate (TNE-4)

A 1000-ml four-necked flask equipped with a thermometer and a cooling tube was charged with 200 g of thiolactic acid, 216 g of 1-ethoxy-2-propanol and 2.1 g of 95% sulfuric acid, followed by stirring at 100°C for 3 hours. During the reaction, the pressure was slightly reduced by means of an aspirator connected with an upper end of the cooling tube, and thereby water that occurred with progress of the reaction was distilled away. After the completion of the reaction, part of the reaction liquid was sampled and analyzed by HPLC to determine the yield, which was found to be 39% from calculation of the concentration of the objective compound in the reaction liquid.
Subsequently, the reaction liquid was directly concentrated and distilled under reduced pressure (b.p. 101°C / 2.4 kPa). This purification provided 224 g of 2-ethoxy-1-methylethyl thiolactate (TNE-4).
The results of TNE-4 identification are given below.
GC-MS: 178, 147, 132, 117, 72
IR (Zn-Ce): 2978, 1732, 1452, 1173, 1112, 1061, 1024

[Example 1]

A permanent wave first liquid was prepared as described below using the alkoxyalkyl mercaptocarboxylate (TGE-1) obtained in Synthetic Example 1. A permanent wave second liquid was prepared as described below. Hair was

permanent waved with these liquids, and the wave efficiency was obtained.

(Preparation of permanent wave first liquid)

22 mmol of the alkoxyalkyl mercaptocarboxylate (TGE-1) obtained in Synthetic Example 1, 10 g of propylene glycol, 0.2 g of disodium edetate (EDTA-2Na) and 1 g of polyoxyethylene stearyl ether were mixed with monoethanolamine and purified water to achieve a desired pH (pH 6.5, 7.5 or 9). The mixing was performed such that the total amount would be 100 g. A permanent wave first liquid was thus prepared.
The agent contained TGE-1 in an amount of 2% in terms of reducing power of thioglycolic acid.

(Preparation of permanent wave second liquid)

5 g of sodium bromate and 95 g of purified water were mixed together to give a permanent wave second liquid.

(Permanent wave processing)

The wave efficiency was evaluated by a Kilby method as described in Fragrance Journal Extra Edition (1984, No. 5, P. 442). A liquid containing cysteamine hydrochloride was used as the comparative agent.
Chinese hair (approximately 20 cm long) was fixed to a Kilby apparatus and was soaked in the permanent wave first liquid heated to 35°C over a period of 20 minutes. Thereafter, the hair was lightly towel dried such that the first liquid would not drip. The hair was then given the bromate-based permanent wave second liquid, and was allowed to stand at 25°C for 10 minutes. After the completion of the treatment with the second liquid, the hair was rinsed with running water and was removed from the Kilby apparatus, followed by drying. The dried hair was measured and the wave efficiency was determined from the following equation. The results are shown in Table 1.

$$\text{Wave efficiency (\%)} = 100-[100 \times (B-A)] \div (C-A)$$

wherein:

A: Distance between the first and sixth rods of the Kilby apparatus (actual measurement value between the central points of the rods)
B: Length of six waves of curled hair
C: Length of straightened six waves of curled hair

[Examples 2 to 13]

A permanent wave first liquid was prepared as described in Example 1, except that TGE-1 was replaced with the alkoxyalkyl mercaptocarboxylate indicated in Table 1. The permanent wave first liquid contained the alkoxyalkyl mercaptocarboxylate in an amount of 2% in terms of reducing power of thioglycolic acid.
Hair was permanent waved and the wave efficiency was obtained by the procedure of Example 1 with use of the permanent wave first liquid prepared as above and the permanent wave second liquid used in Example 1. The results are given in Table 1.

[Comparative Example 1]

A permanent wave first liquid was prepared as described in Example 1, except that TGE-1 was replaced with the cysteamine hydrochloride indicated in Table 1. The permanent wave first liquid contained the cystamine hydrochloride in an amount of 2% in terms of reducing power of thioglycolic acid.
Hair was permanent waved and the wave efficiency was obtained by the procedure of Example 1 with use of the permanent wave first liquid prepared as above and the permanent wave second liquid used in Example 1. The results are given in Table 1.

Table 1

| | Compound used | Wave efficiency | | |
|---|---|---|---|---|
| | | pH 6 | pH 7.5 | pH 9 |
| Example 1 | TGE-1 | 83% | 85% | 85% |
| Example 2 | TGE-2 | 84% | 87% | 86% |
| Example 3 | TGE-3 | 79% | 79% | 82% |
| Example 4 | TGE-4 | 75% | 76% | 76% |
| Example 5 | TGE-5 | 70% | 70% | 71% |
| Example 6 | TGE-6 | 63% | 67% | 65% |
| Example 7 | TGE-7 | 79% | 81% | 81% |
| Example 8 | TGE-8 | 80% | 82% | 83% |
| Example 9 | TGE-9 | 75% | 75% | 76% |
| Example 10 | TNE-1 | 76% | 78% | 79% |
| Example 11 | TNE-2 | 73% | 75% | 76% |
| Example 12 | TNE-3 | 73% | 73% | 72% |
| Example 13 | TNE-4 | 65% | 68% | 68% |
| Comparative Example 1 | Cysteamine hydrochloride | 52% | 65% | 73% |

The permanent hair processing agents containing the alkoxyalkyl mercaptocarboxylate of this invention achieved stable wave efficiencies in the neutral to acidic pH range as well as in the alkaline pH range.

On the other hand, Comparative Example 1 that employed the permanent hair processing agent containing the cysteamine hydrochloride resulted in drastic deterioration of wave efficiency with the pH lowering to a neutral to acidic region.

## Claims

1. A method for permanent hair processing comprising (1) applying to hair a reducing agent consisting of at least one of compounds represented by the following formula (1) and/or formula (2):

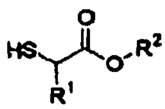

(1)

wherein $R^1$ is a hydrogen atom or an alkyl group of 1 to 5 carbon atoms, $R^2$ is an alkoxyalkyl group having a total of 3 to 15 carbon atoms, and the alkylene portion of $R^2$ may contain an ether bond(s);

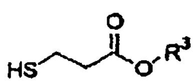

(2)

wherein $R^3$ is an alkoxyalkyl group having a total of 3 to 15 carbon atoms, and the alkylene portion of $R^3$ may contain an ether bond(s), at a pH of 6 to 8.5,
(2) allowing the hair wetted with the agent to stand,
(3) oxidising and tightening the hair with a composition containing an oxidant, and
(4) rinsing, shampooing and drying the hair.

2. The method for permanent hair processing according to claim 1, wherein the agent contains at least one compound of the formula (1) in which $R^1$ is a hydrogen atom, a methyl group, an ethyl group or a propyl group, and $R^2$ is an

alkoxyalkyl group including an alkoxy group of 1 to 4 carbon atoms and an alkylene group of 1 to 8 carbon atoms (the alkylene group of $R^2$ may contain an ether bond(s)).

3. The method for permanent hair processing according to claim 1, wherein the agent contains at least one compound of the formula (2) in which $R^3$ is an alkoxyalkyl group including an alkoxy group of 1 to 4 carbon atoms and an alkylene group of 1 to 8 carbon atoms (the alkylene group of $R^3$ may contain an ether bond(s)).

4. The method for permanent hair processing according to claim 1, wherein the agent contains the compound(s) represented by the formula (1) and/or formula (2) in an amount of 0.5 to 30% in terms of reducing power of thioglycolic acid.

5. The method for permanent hair processing according to claim 1 or 4, wherein the agent contains two or more compounds represented by the formula (1) and/or formula (2).

**Patentansprüche**

1. Verfahren zur permanenten Haarbearbeitung, welches (1) das Auftragen eines Reduktionsmittels, das aus mindestens einer der Verbindungen der folgenden Formel (1) und/oder Formel (2) besteht:

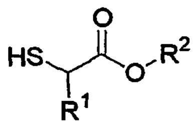
(1)

worin $R^1$ ein Wasserstoffatom oder eine Alkylgruppe mit 1 bis 5 Kohlenstoffatomen ist, $R^2$ eine Alkoxyalkylgruppe mit insgesamt 3 bis 15 Kohlenstoffatomen ist und der Alkylenteil von $R^2$ eine oder mehrere Etherbindungen aufweisen kann;

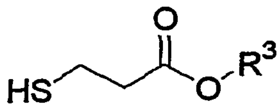
(2)

worin $R^3$ eine Alkoxyalkylgruppe mit insgesamt 3 bis 15 Kohlenstoffatomen ist und der Alkylenteil von $R^3$ ein oder mehrere Etherbindungen enthalten kann,
bei einem pH-Wert von 6 bis 8,5 auf Haar,
(2) das Stehenlassen des mit dem Mittel nassgemachten Haares,
(3) das Oxidieren und Festigen des Haares mit einer ein Oxidationsmittel enthaltenden Zusammensetzung, und
(4) das Spülen, Shampoonieren und Trocknen des Haares.

2. Verfahren zur permanenten Haarbearbeitung nach Anspruch 1, wobei das Mittel mindestens eine Verbindung der Formel (1) enthält, worin $R^1$ ein Wasserstoffatom, eine Methylgruppe, eine Ethylgruppe oder eine Propylgruppe ist und $R^2$ eine Alkoxyalkylgruppe ist, die eine Alkoxygruppe mit 1 bis 4 Kohlenstoffatomen und eine Alkylengruppe mit 1 bis 8 Kohlenstoffatomen (wobei die Alkylengruppe von $R^2$ eine oder mehrere Etherbindungen aufweisen kann) umfasst.

3. Verfahren zur permanenten Haarbearbeitung nach Anspruch 1, wobei das Mittel mindestens eine Verbindung der Formel (2) enthält, worin $R^3$ eine Alkoxyalkylgruppe ist, die eine Alkoxygruppe mit 1 bis 4 Kohlenstoffatomen und eine Alkylengruppe mit 1 bis 8 Kohlenstoffatomen (wobei die Alkylengruppe von $R^3$ eine oder mehrere Etherbindungen enthalten kann) umfasst.

4. Verfahren zur permanenten Haarbearbeitung nach Anspruch 1, wobei das Mittel die Verbindung(en) der Formel (1) und/oder der Formel (2) in einer Menge von 0,5 bis 30%, bezogen auf die Reduktionskraft von Thioglycolsäure, enthält.

**5.** Verfahren zur permanenten Haarbearbeitung nach Anspruch 1 oder 4, wobei das Mittel zwei oder mehr Verbindungen der Formel (1) und/oder der Formel (2) enthält.

## Revendications

**1.** Procédé pour la transformation permanente de cheveux comprenant (1) l'application aux cheveux d'un agent réducteur constitué d'au moins un des composés représentés par la formule (1) ou la formule (2) suivantes :

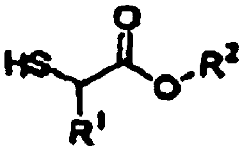

(1)

où $R^1$ est un atome d'hydrogène ou un groupe alkyle ayant de 1 à 5 atomes de carbone, $R^2$ est un groupe alcoxyalkyle ayant un total de 3 à 15 atomes de carbone, et la portion alkylène de $R^2$ peut contenir une(des) liaison(s) éther ;

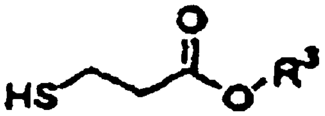

(2)

où $R^3$ est un groupe alcoxyalkyle ayant un total de 3 à 15 atomes de carbone, et la portion alkylène de $R^3$ peut contenir une(des) liaison(s) éther, à un pH de 6 à 8,5,
(2) le laisser reposer des cheveux mouillés avec l'agent,
(3) l'oxydation et la fixation des cheveux avec une composition contenant un oxydant, et
(4) le rinçage, le shampoing et le séchage des cheveux.

**2.** Procédé pour la transformation permanente de cheveux selon la revendication 1, dans lequel l'agent contient au moins un composé de la formule (1) dans laquelle $R^1$ est l'atome d'hydrogène, le groupe méthyle, le groupe éthyle ou le groupe propyle, et $R^2$ est un groupe alcoxyalkyle comprenant un groupe alcoxy ayant de 1 à 4 atomes de carbone et un groupe alkylène ayant de 1 à 8 atomes de carbone (le groupe alkylène de $R^2$ peut contenir un(des) liaison(s) éther).

**3.** Procédé pour la transformation permanente de cheveux selon la revendication 1, dans lequel l'agent contient au moins un composé de la formule (2) dans laquelle $R^3$ est un groupe alcoxyalkyle comprenant un groupe alcoxy ayant de 1 à 4 atomes de carbone et un groupe alkylène ayant de 1 à 8 atomes de carbone (le groupe alkylène de $R^3$ peut contenir une(des) liaison(s) éther).

**4.** Procédé pour la transformation permanente de cheveux selon la revendication 1, dans lequel l'agent contient le (les) composé(s) représenté(s) par la formule (1) et/ou la formule (2) dans une quantité de 0,5 à 30 % en termes de puissance réductrice d'acide thioglycolique.

**5.** Procédé pour la transformation permanente de cheveux selon la revendication 1 ou 4, dans lequel l'agent contient deux ou plusieurs composés représentés par la formule (1) et/ou la formule (2).

[Fig.1]

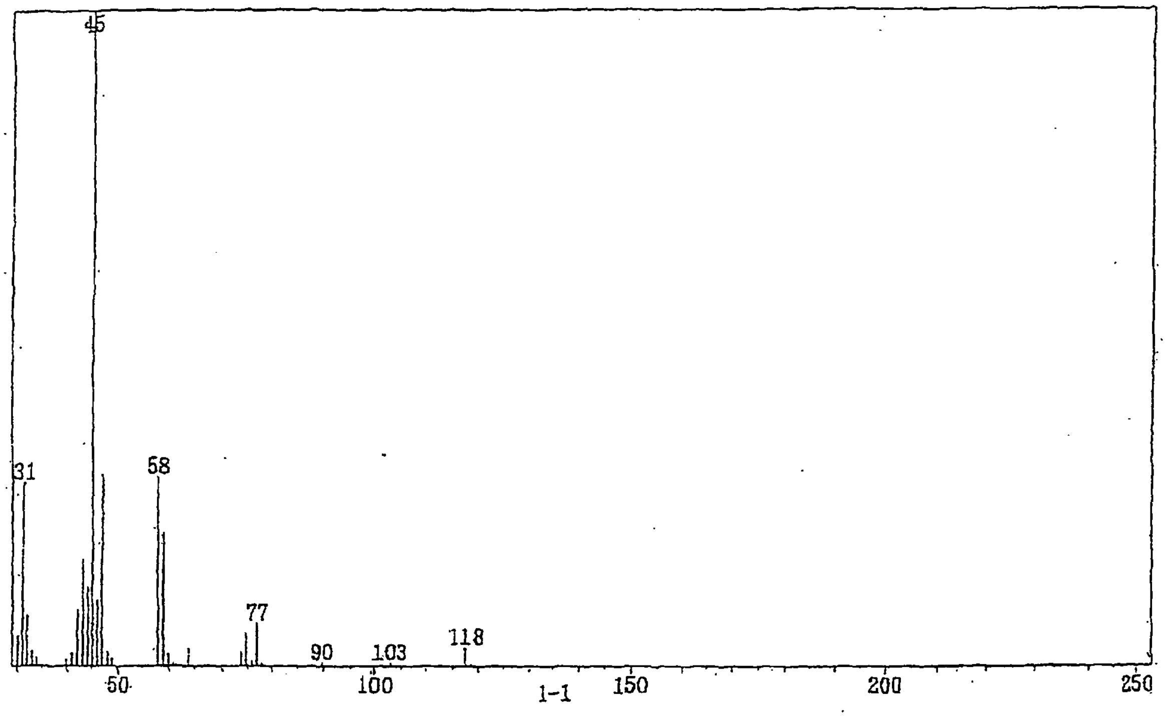

[Fig.2]

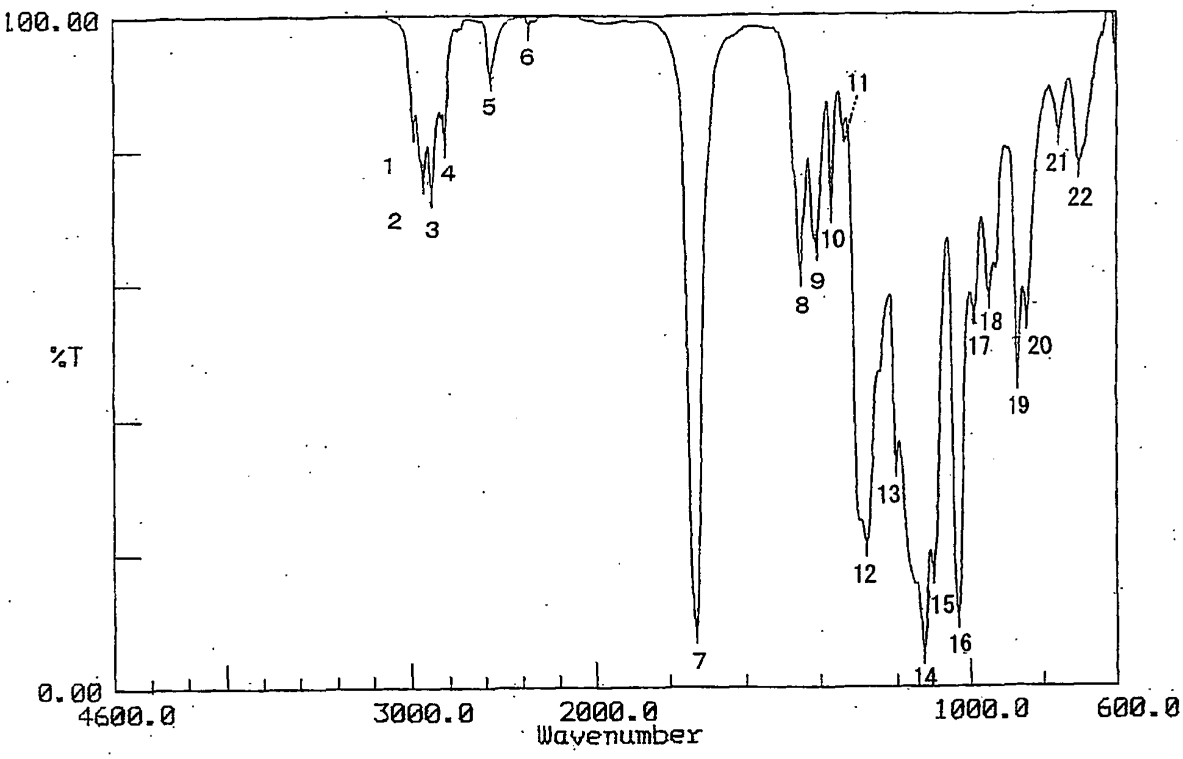


Condition
upper 100.00 lower 0.00 depth 1.00

Peak table

| | | |
|---|---|---|
| 1: 2985.11( 83.8) | 2: 2932.06( 76.0) | 3: 2889.63( 74.4) |
| 4: 2821.15( 81.4) | 5: 2566.52( 91.2) | 6: 2363.01( 98.6) |
| 7: 1732.23( 8.7) | 8: 1451.56( 61.8) | 9: 1408.16( 65.5) |
| 10: 1368.62( 71.2) | 11: 1339.68( 83.4) | 12: 1275.06( 21.6) |
| 13: 1198.87( 33.7) | 14: 1123.64( 5.7) | 15: 1099.52( 17.7) |
| 16: 1033.94( 11.0) | 17: 991.50( 56.0) | 18: 949.06( 58.4) |
| 19: 865.15( 46.2) | 20: 841.04( 55.2) | 21: 756.16( 82.7) |
| 22: 700.22( 77.7) | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H08291031 A
- JP 2000507272 A
- JP 2003528901 A
- JP S50111242 A
- US 3880174 A


### Non-patent literature cited in the description


- *Fragrance Journal Extra Edition,* 1984, 442